# EUROPEAN PATENT APPLICATION

(11) **EP 2 106 798 A1**
(43) Date of publication of application: **07.10.2009**
(21) Application number: 08153937.1
(22) Date of filing: 02.04.2008
(51) Int. Cl.: A61K 31/7016, A61P 19/04, A61P 19/02

(54) **Sulfated unsaturated disaccharidic chondroitin sulfate in connective tissue protection and repair**

(71) Applicant: Nestec S.A., 1800 Vevey (CH)
(72) Inventor: Ameye, Laurent, 1000 Lausanne 26 (CH); Austin, Sean, Christopher, 1066 Epalinges (CH); Tissot-Favre, Delphine, Webster Groves, MO 63119 (US)
(74) Representative: Marquardt, Ulf

(57) **Abstract**

The present invention generally relates to the protection and repair of connective tissues. In particular, the present invention relates to the use of chondroitin sulfates and its derivatives in connective tissue protection and repair.

The present inventors have found that unsaturated disaccharidic chondroitin sulfate can be used for the preparation of a composition to develop, protect and/or repair connective tissues. This allows it for example to treat or prevent osteoarthritis or rheumatoid arthritis, and/or the symptoms thereof as well as to treat or prevent signs of skin ageing such as the appearance of wrinkles effectively.

## Description

The present invention generally relates to the protection and repair of connective tissues. In particular, the present invention relates to the use of chondroitin sulfates and its derivatives in connective tissue protection and repair.

Osteoarthritis (OA) is one of the most prevalent and disabling chronic diseases affecting the elderly. Its most prominent feature is the progressive destruction of articular cartilage which results in impaired joint motion, severe pain and, ultimately, disability. Its high prevalence and its moderate to severe impact on daily life pose a significant public health problem. The prevalence of OA of the knee in Western Europe has been estimated as 18-25% in men and 24-40% in women between ages 60-79 in Holland and 28-34% in Spain. There are estimates of 100 million people with OA in the European Union. The estimated direct cost of OA in France in 2001 was 1.64 billion Euros. In the US, the burden of OA was 69.9 million people in 2001.

Today, a cure for OA remains elusive and the management of OA is largely palliative focusing on the alleviation of symptoms. Current recommendations for the management of OA include a combination of non-pharmacological interventions (weight loss, education programs, exercise, etc) and pharmacological treatments (paracetamol, nonsteroidal antiinflammatory drugs -NSAIDs-, etc).

Chondroitin sulfate (CS) is among the most used therapies for OA. It is ingested orally but depending on the country, it is sold as a drug or as a dietary supplement. Together with glucosamine, the market size for CS is estimated to be 800 millions US$ per year. The ability of CS to decrease knee pain and improve knee function in osteoarthritic patients has been demonstrated in multiple clinical trials and confirmed in meta-analyses. According to the recommendations from the scientific European League against Rheumatism, CS displays the highest level of evidence of efficacy (i.e. meta-analyses of randomized clinical trials -RCTs) for knee OA. Out of 7 double-blind placebo-controlled RCTs lasting from 3 to 24 months and evaluating its symptom-modifying effects on knee OA, 6 concluded that CS was more efficient than placebo in decreasing spontaneous pain and increasing mobility [L'Hirondel JL, Litera Rheumatologica 1992, 14: 77-84; Bucsi L, Poor G,Osteoarthritis Cartilage 1998, 6 Suppl A: 31-36; Uebelhart D, et al., Osteoarthritis Cartilage 1998, 6 Suppl A: 39-46; Pavelka K, et al., Litera Rheumatologica 1999, 24: 21-30; Bourgeois P, et al., Osteoarthritis Cartilage 1998, 6 Suppl A: 25-30; Uebelhart D, et al., IOsteoarthritis Cartilage 2004, 12: 269-276.]. 800 and 1200 mg once a day were more effective on pain and mobility than placebo or 200 mg. No statistical significant difference on mobility were found between 1200 mg of CS once a day and 400 mg three times a day and both treatments were superior to placebo. Two 3 month-long periods of treatment in a year with 800 mg once a day demonstrated lasting effects by improving mobility compared to placebo. Effects were not immediate; at least 2 weeks were required before patients could detect a significant difference in pain and mobility. In one trial CS (1000 mg per day) did not improve function better than placebo [Mazieres B, et al., J Rheumatol 2001, 28: 173-181]. In a double-blind RCT, 400 mg of CS 3 times a day was slower than 50 mg of diclofenac 3 times a day in improving the Lesquesne index (a validated index of pain and function) but not in decreasing a visual analogue scale of pain [Morreale P, et al.,. J Rheumatol 1996, 23: 1385-1391]. CS effects persisted longer than diclofenac effects after termination of treatment. The symptom-modifying effects of CS on knee OA are further supported by 3 meta-analyses [Leeb BF, et al., J Rheumatol 2000, 27: 205-211; McAlindon TE, et al., Jama-Journal of the American Medical Association 2000, 283: 1469-1475; Richy F, et al., Arch Intern Med 2003, 163: 1514-1522]. These meta-analyses concluded that CS was superior to placebo, had large effects on osteoarthritic symptoms and was effective on multiple validated instruments of pain and function for OA (Lesquene index, visual analogue scale of pain, visual analogue scale of mobility and responding status).

In addition to its symptom-modifying effects, CS was reported to slow down cartilage destruction in OA patients in 3 trials. These trials used as primary endpoints the joint space loss/width measured on successive X-rays taken several years apart, the current gold-standard surrogate marker for cartilage destruction. In a first one year double-blind placebo-controlled RCT, mean and minimum joint space width and joint space surface area measured on X-rays decreased significantly in the placebo group but not in the 800 mg CS group [Uebelhart D, et al., Osteoarthritis Cartilage 2004, 12: 269-276]. Changes in osteocalcin, keratan sulfate, pyridinoline and deoxypyridinoline biomarkers in that trial suggested that CS affected bone and cartilage metabolism. In a second 2-year double-blind RCT on knee OA, 800 mg once a day prevented joint space narrowing compared to a mean joint space loss of 0.14 mm in the placebo group [Michel BA, et al., Arthritis Rheum 2005, 52: 779-786.]. These results were confirmed in a third 2-year double blind RCT on knee OA enrolling 622 patients (STOPP trial, in press). Finally, in a small placebo-controlled open trial, 10 days of 800 mg of CS once a day significantly increased the viscosity and hyaluronate concentration while decreasing the activities of collagenase, phospholipase A2 and N-acetylglucosaminidase in knee synovial fluids, further supporting the existence of symptom and structure-modifying effects [Ronca F, et al.,Osteoarthritis and Cartilage 1998, 6: 14-21]. The structure-modifying effects of CS are also supported by animal studies. In young rats, 100 and 300 mg/kg body weight/day of chondroitin-6 sulfate (5 and 15 times the clinical dose), starting 2 weeks before injections of bradikinin in the knee, reduced or prevented proteoglycan depletion in cartilage [Omata T, et al., Arzneimittelforschung 1999, 49: 577-581]. In young rabbits, 20 mg/kg body weight/day of CS beginning 11 days prior to chymopapain injection and continuing for 21 days after the injection decreased cartilage damages and proteoglycan loss [Uebelhart D, et al., Osteoarthritis and Cartilage 1998, 6: 6-13].

Taken together, the above summarized prior art allows the conclusion that today, chondroitin sulfate is one of the few molecules able to improve symptoms and to slow down articular cartilage destruction. However, although efficacious, its effects are slow (i.e. up to 2 months are required to reach full symptom relief) and partial (i.e. the patients feel better but don't feel good).

Consequently, it was the object of the present invention to identify specific chondroitin sulfates that have superior effects and a higher efficacy compared to the chondroitin sulphates generally known and employed in the art. Hence, the present invention aims to provide a more efficient therapy to osteoarthritic patients and patients suffering from other connective tissue disease.

The present inventors have investigated saccharides of CS and have demonstrated that a very specific group of them, namely unsaturated disaccharidic chondroitin sulfate, in particular 4-sulfated unsaturated disaccharidic chondroitin sulfate, was particularly effective for the purpose of the present invention.

Notably, no other tested saccharides of CS, not any of the tested saturated disaccharides nor commercial CS were able to decrease the destruction of glycosaminoglycans (GAG) in cartilage in the tested assay to a similar extent than the compounds claimed.

This particular high efficacy of sulfated unsaturated disaccharidic chondroitin sulfate for the purpose of the present invention is new and unexpected. The present inventors were hence surprised to see that the object of the present invention could be achieved by a use in accordance with claim 1.

One embodiment of the present invention is unsaturated disaccharidic chondroitin sulfate for developing, protecting and/or repairing connective tissues.

Among several different chondroitin disaccharides and several higher molecular weight forms of chondroitin sulfate, 4-sulfated unsaturated chondroitin sulfate disaccharide (Δdi4S) was by far the most effective compound in slowing down cytokine-induced cartilage destruction *in vitro.* Sulfatation, ideally in position-4 of the N-acetyl-galactosamine residue, and unsaturation at between positions 4 and 5 of the glucoronic acid ring 2 appear to be beneficial for this remarkable efficacy as structurally closely related compounds did not reduce cytokine-induced cartilage destruction as the 4-sulfated unsaturated disaccharidic chondroitin sulfate of the present invention. Regular commercial chondroitin sulfate did not have any significant effect.

In parallel, in a rat intestinal loop model, the bioavailability of chondroitin sulfate was shown to increase if the molecular weight of the tested molecule decreased. Hence, it was shown that 4-sulfated unsaturated chondroitin sulfate disaccharide is also more bioavailable than regular high molecular weight chondroitin sulfate.

This invention thus provides, for example, a more efficacious and bioavailable form of chondroitin sulfate compared to regular commercial high molecular weight chondroitin sulfate.

Because of this, the compounds of the present invention will provide a higher pain relief and a better protection against cartilage destruction to OA patients than the chondroitin sulfates of the prior art. This finding is hence a significant advance in a therapeutic area where no intervention besides prosthesis surgery is able to decrease osteoarthritic pain sufficiently, to allow the patients to feel good and where few intervention are suggested as being able to slow down cartilage destruction.

Consequently, one embodiment of the present invention is the use of at least one sulfated unsaturated disaccharidic chondroitin sulfate for the preparation of a composition to protect and/or repair connective tissues. Another embodiment is the use of at least one sulfated unsaturated disaccharidic chondroitin sulfate for the preparation of a composition to treat and/or prevent the symptoms of connective tissues diseases.

Connective tissues are one of the four types of tissue in traditional classifications (the others being epithelial, muscle, and nervous tissue.) Connective tissues are typically tissues that are involved in structure and support and are characterized by the presence of an important extracellular organic matrix.

Cartilage is a typical connective tissue, for example.

Chondroitin sulfate is a sulfated glycosaminoglycan (GAG) composed of a chain of alternating sugars (N-acetylgalactosamine and glucuronic acid). It is often found attached to proteins as part of a proteoglycan. A chondroitin chain can have over 100 individual sugars. Each monosaccharide may be left unsulfated, sulfated once, or sulfated twice. Most commonly the hydroxyls of the 4 and 6 positions of the N-acetylgalactosamine are sulfated.

The sulfated unsaturated disaccharidic chondroitin sulfate of the present invention is preferably selected from the group consisting of 4-sulfated unsaturated disaccharidic chondroitin sulfate (Δdi4S), 6-sulfated unsaturated disaccharidic chondroitin sulfate (Δdi6S) and non-sulfated unsaturated disaccharidic chondroitin sulfate (Δdi0S).

The composition that may be prepared by the use of the present invention can be any composition that can be used to administer sulfated unsaturated disaccharidic chondroitin sulfate to a human or pet. Preferably, it is a pharmaceutical composition, a beverage, a food product, a food supplement and/or a nutraceutical.

The composition may preferably be selected from the group consisting of milk powder based products; instant drinks; ready-to-drink formulations; nutritional powders; milk-based products, in particular yoghurts or ice cream; cereal products; biscuits; cereal bars; beverages; water; coffee; cappuccino; tea; fruit juices; malt drinks; chocolate flavoured drinks; culinary products; soups; confectionary products; chocolates; topical creams, suppositories, tablets, syrups, and formulations for transdermal applications.

In one aspect of the present invention the composition prepared by the use of the present invention is used to treat or prevent connective tissue diseases. Typical connective tissue diseases can be both inherited and environmental and comprise the Marfan syndrome, scurvy, Ehlers-Danlos syndrome, Loeys-Dietz syndrome, Pseudoxanthoma elasticum, Systemic lupus erythematosus, Osteogenesis imperfecta (brittle bone disease), Fibrodysplasia ossificans progressive, Spontaneous pneumothorax, and/or Sarcoma.

Two connective tissue diseases that are most preferably treated or prevented by the use of the present invention are osteoarthritis and rheumatoid arthritis.

The composition prepared by the use of the present invention may also be used to treat or prevent the symptoms of osteoarthritis or rheumatoid arthritis, such as for example pain and impaired mobility.

It may also be used for cosmetic reasons, for example to treat or prevent signs of skin ageing such as the appearance of wrinkles.

The present inventors have found that sulfated unsaturated disaccharidic chondroitin sulfate can in particular be used to balance anabolic and catabolic activities of chondrocytes and or to slow down or stop cartilage destruction, in particular articular cartilage destruction.

Because of its high efficacy and bioavailability the composition comprising the sulfated unsaturated disaccharidic chondroitin sulfate can also be used to provide pain relief, particularly in OA patients.

The composition prepared by the use of the present invention may also be used to improve mobility, in particular to improve joint function.

The sulfated unsaturated disaccharidic chondroitin sulfate may be used alone or in combination with other compounds. The compositions prepared by the use of the present invention may also contain combinations of the sulfated unsaturated disaccharidic chondroitin sulfates disclosed herein and/or may also contain the chondroitin sulfates that are presently known in the art.

The compositions may also further comprise at least one aggrecan, at least one collagen, at least one chondroitin sulfate and/or at least one glucosamine or fractions thereof.

Along with Type-II collagen, aggrecan forms a major structural component of cartilage, particularly articular cartilage. Aggrecan consists of two globular structural domains at the N-terminal end and one globular domain at the C-terminal end, separated by a large domain heavily modified with glycosaminoglycans. The linker domain between the N-terminal globular domains, called the interglobular domain, is highly sensitive to proteolysis. Such degradation has been associated with the development of arthritis. Proteases capable of degrading aggrecans are called aggrecanases, and they are members of the ADAMTS (A Disintegrin And Metalloprotease with ThromboSpondin motifs) protein family. Consequently, the efficacy of the composition prepared by the use of the present invention can be increased, if the composition further comprises at least one ADAMTS inhibitor, in particularly an aggrecanase inhibitor and/or a matrix metalloproteinase inhibitor.

Dependant on the intended use of the composition prepared in accordance with the present invention it may further comprise at least one other compounds, for example at least one compound selected from the group consisting of a pain relieving agent, a stabilizing agent, a flavouring agent, a colouring agent, a lubricant, an anti-inflammatory agent, an inhibitor of angiogenesis, a chondroprotective agent, a bone anti-resorptive agent and/or an agent increasing bone formation.

It may be desired that the composition prepared according to the present invention releases its active compounds slowly in a controlled manner over a long period of time. This way, frequent administrations can be avoided and a long term effect can be achieved. For this purpose the composition may be prepared as a sustained release formulation, for example. The active compounds can be for example appropriately encapsulated so that they are slowly released in amounts that correspond to the immediate demand.

The composition may also comprise a carrier. Carriers ensure for example good dosability and the possibility of long storage times. Any carrier known in the art is applicable to the present invention and its selection will depend on the composition and its intended use.

The composition is intended for human or animals, in particular pets and/or companion animals. With respect to animals, animals expected to have a long lifespan are preferred. The compositions of the present invention can be used for example to ensure a good mobility for a long time. Cats and dogs are particularly preferred.

The compositions prepared according to the present invention may be applied to any age group. The composition is intended for example for infants, children, adolescents, adults and/or the elderly. However, since connective tissue protection and repair is rather an issue of the adult or elderly population, these age groups are preferred.

Development of connective tissue may be equally an issue for all age groups, for example after an injury. In particular for infants, the development of connective tissue is important, for example, to ensure the generation of a strong bone-cartilage system.

The dosage of the sulfated unsaturated disaccharidic chondroitin sulfate in the composition of the present invention will depend crucially for example on the nature of the composition, the condition to be treated, the age, size, health status and gender of the patient and/or on other medicamentation that might be taken simultaneously, only to name a few. The dose is hence not particularly limited and any effective dose is applicable for the purpose of the present invention. An effective dose can easily be determined by medical personnel or by a nutritionist.

However, in general it is preferred that the 4-sulfated unsaturated chondroitin sulfate is present in the composition in an amount of about 100ng-1g/g dry mass of the composition, preferably about 1mg -1g/g dry mass of the composition, even more preferred about 200mg-1g/g dry mass of the composition.

In terms of daily ingestion it is preferred if the 4-sulfated unsaturated chondroitin sulfate is to be administered in a daily amount of about 1ng-100mg /kg body weight of the subject to be treated, preferably about 1mg-80mg /kg body weight of the subject to be treated, even more preferred about 5mg-50mg /kg body weight of the subject to be treated.

The effective dose of sulfated unsaturated disaccharidic chondroitin sulfate may for example lie between about 800 mg and 1200 mg a day for an adult patient, given at once or, for example split in 3 single dosages during the day.

It is clear to those skilled in the art that they can freely combine all features of the present invention described herein without departing from the scope of the invention as disclosed. In particular it all features described for the use of the present invention can be applied to the unsaturated disaccharidic chondroitin sulfate of the present invention.

Further advantages and features of the present invention will be apparent from the following examples and figures.

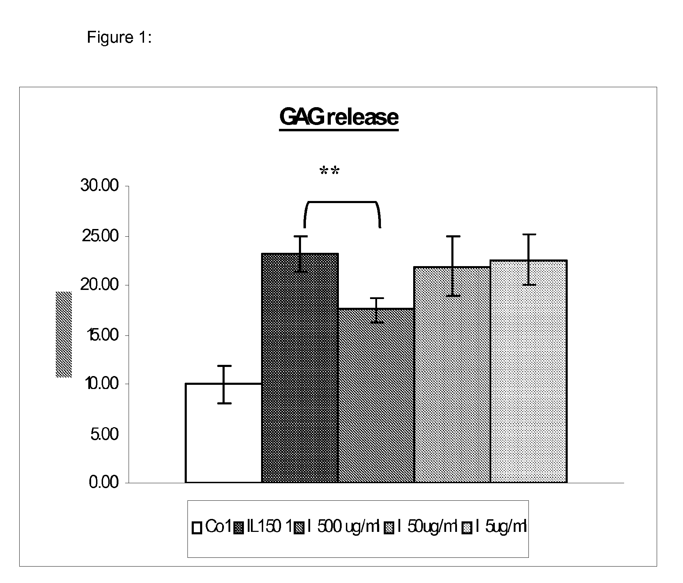

Figure 1: Effect of Δdi4S on IL1 β-induced GAG release. After being loaded with radioactivity, cartilage explants were treated for 72 hours with DMEM only (in yellow) or with 50ng/ml of IL1β only (in purple) or with IL1β and ΔDi4S (in blue). The dose range for metabolite I was: 500 - 50 - 5 µg/ml. Data are expressed as the percentage of radioactivity release (mean of 6 culture wells ± standard deviation), with percentage of radioactivity release = supernatant radioactivity / (supernatant radioactivity + explants radioactivity). The percentage of radioactivity released is used as a surrogate marker for GAG release; (**: p<0.001).

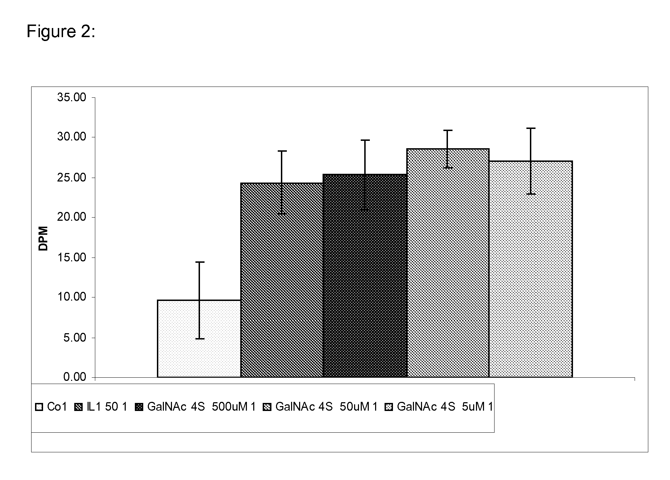

Figure 2: Effect of 4 sulfated N-acetylgalactosamine (GalNAc 4S) on IL1 β-induced GAG release. After being loaded with radioactivity, cartilage explants were treated for 72 hours with DMEM only (in yellow) or with 50ng/ml of IL1β only (in purple) or with IL1β and GalNac 4S (in blue). The dose range for GalNac 4S was: 500 - 50 - 5 µg/ml. Data are expressed as the percentage of radioactivity release (mean of 6 culture wells ± standard deviation), with percentage of radioactivity release = supernatant radioactivity / (supernatant radioactivity + explants radioactivity). The percentage of radioactivity released is used as a surrogate marker for GAG release). GalNac 4S had no effect on the GAG release.

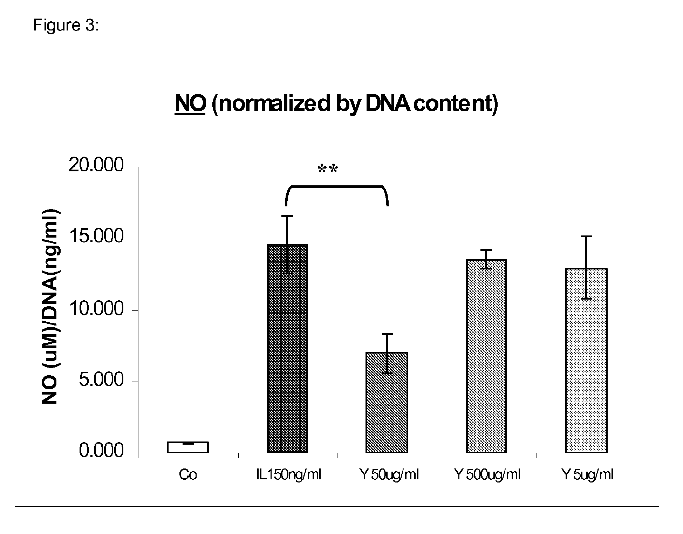

Figure 3: Effect of Δdi4S on IL1 β-induced NO production. Cartilage explants were treated for 72 hours with DMEM only (in yellow) or with 50ng/ml of IL1β only (in red) or with IL1β and ΔDi4S (in blue). The dose range for Δdi4S is: 500 - 50 - 5 µg/ml. Data are presented as the amount of NO in uM in supernatants divided by the amount of DNA in explants in ng/ml (means of 6 culture wells ± standard deviation; **: p<0.001)

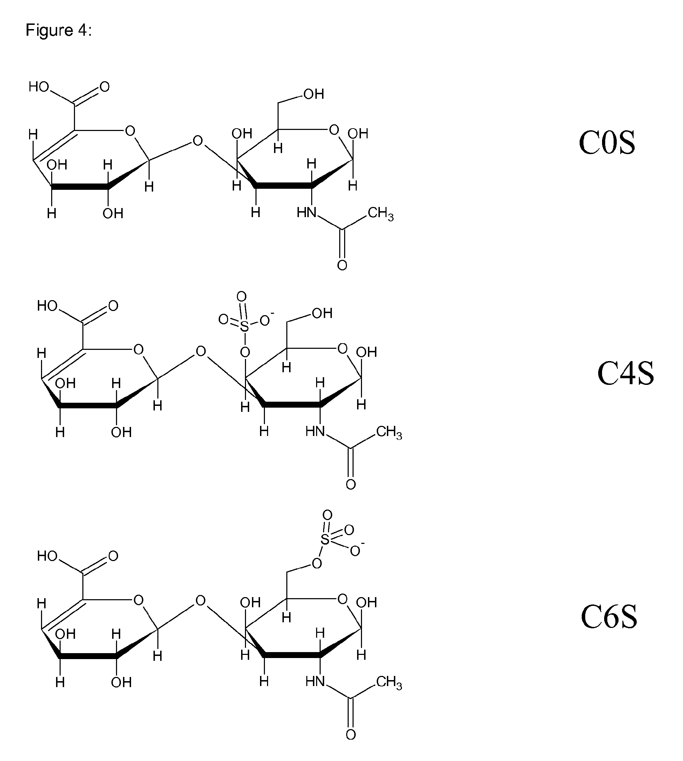

Figure 4: Shown are particular preferred chondroitin sulfates of the present invention, namely 4-sulfated unsaturated disaccharidic chondroitin sulfate (Δdi4S) (C4S), 6-sulfated unsaturated disaccharidic chondroitin sulfate (Δdi6S) (C6S) and non-sulfated unsaturated disaccharidic chondroitin sulfate (Δdi0S) (C0S)

### Examples:

Osteoarthritis is a disease characterized by a slow destruction of articular cartilage. This cartilage destruction is due to an imbalance between the anabolic and catabolic activity of chondrocytes. The chondrocyte is the unique cell type present in cartilage and is responsible for the maintenance of the cartilage extracellular matrix. In osteoarthritis, catabolism is increased and is responsible for the cartilage loss. The extracellular matrix of cartilage is composed of 2 main molecules: type II collagen and aggrecan. While collagen is mainly digested by matrix metalloproteinases (MMPs), aggrecan can be degraded both by MMPs and another class of enzymes called aggrecanases.

It was investigated here whether normal commercially available chondroitin sulphate and different low molecular weight forms of chondroitin sulphate including disaccharides could inhibit the degradation of aggrecan in cultured explants of articular cartilage. Explants from healthy bovine articular cartilage were put in culture and loaded with S³⁵. S³⁵ is incorporated in the glycosaminoglycan chains of the aggrecan molecules of the cartilage and its release in the media is considered as a surrogate marker of glycosaminoglycan release and aggrecan catabolism. However, explants from healthy bovine articular cartilage naturally display a very low catabolic activity which is not ideal to test the anti-catabolic activity of potential bioactives. To improve the sensitivity of the assay, the explants were cultured in presence of IL1β, a pro-inflammatory cytokine, known to act as a potent inducer of MMP and aggrecanase activities. The different low molecular weight forms of chondroitin sulphate were added to the culture media together with IL1β to see whether they can decrease the IL1β-induced aggrecan catabolism. In addition, the effects of the low molecular weight forms of chondroitin sulphate on IL1β-induced NO production were also investigated. Nitric oxide is a reactive oxygen species known to participate in the progression of osteoarthritis.

### Experimental procedure:

The articular cartilage explants were dissected out of the metacarpophalangeal joint of old cows (8-10 years). The skin was removed from the feet. The articulation was opened transversally. Intraarticular ligaments were transected. Full thickness slices of cartilage were dissected out and put into a Petri dish containing DMEM supplemented with 20% FBS (fetal bovine serum albumin) and antibiotics (penicillin, streptomycin and gentamycin). Under the hood, similar quantities of cartilage were distributed between the wells of a 24-wells plate containing 500µl of medium (DMEM + 20% FBS, 1% penicillin/streptomycin, 0.1% gentamycin) per well. Finally, the plates were put in the incubator (37°C / 5% CO₂).

Five days after their harvest, the explants were divided into 5 different treatment conditions:
*1- negative control:* the wells contain only culture medium.
*2- positive stimulatory control:* Interleukin 1β (IL1β) is added to the media at the concentration of 50ng per ml. This cytokine is used to stimulate the catabolism and to induce inflammation.
   3/4/5 - *tested compounds:* these conditions test the effects of three concentrations of a metabolite of chondroitin sulfate in the presence of IL1β.
   The explants undergo these treatments for 72 hours in the incubator (37°C, 5% CO₂).

The following protocol is based on Campbell et al., Arch Biochem Biophys 234(1): 275-289 and Hascall VC et al., Arch Biochem Biophys 224(1): 206-223. 48 hours after the harvest of the explants, Sulphur 35 (35S) was added to the medium to be incorporated into newly synthesized polysulfated glycosaminoglycans within the extracellular matrix of the explants. After 72 hours, several washes were performed to eliminate the non-incorporated radioactivity. The explants were then treated for 72hours, after which the supernatants were collected and the amount of radioactivity released in the medium was measured with a beta counter, as a marker of GAG catabolism. The radioactivity present in the explants was also measured and used to normalize the amount of radioactivity released into the media. The radioactivity was expressed in DPM (degradations per minute) and the GAG release is assessed by the following formula:
GAG release = radioactivity in the supernatants / (radioactivity in the supernatants + radioactivity in the explants)

For NO measurements a protocol based on Gilliam et al., Anal Biochem 212(2): 359-365 was used . Briefly, 50 µl of the culture supernatant (SN) were mixed with an equal volume of sulfanilamide solution (in phosphoric acid) in a 96-well plate. Subsequently, 50 µl of Naphtyl-ethylene-diamine solution were added to each well. After ten minutes, absorbance was measured at 538nm. To quantify the amount of NO in the supernatants, a standard curve was prepared using sodium nitrite (0 - 60µM).

### Results:

The stimulation by 50 ng/ml of interleukin 1 beta (IL1β) increased the basal GAG release in the media by a factor included between 1.8 and 4 (mean = 2.5 and median=2.53). Compared to IL1β alone, delta UA--> GalNAc 4S (ΔDi4S) at a concentration of 500µg/ml significantly (p<0.05) decreased the amount of GAG released in the supernatants in 4 independent experiments (see results from one representative experiment in Figure 1). Other saccharides that are closely related structurally to Δdi4S, at the same concentration, did not have these effects (see Figure 2 and Table 1).

The stimulation by 50ng/ml of IL1β increased the NO production in the media by a factor included between 2.9 and 190 (mean = 39.64 and median = 24.98). Compared to IL1β alone, delta UA--> GalNAc 4S at a concentration of 500µg/ml significantly (p<0.05) decreased the amount of NO in the supernatants in the 4 experiments (see Figure 3 for results obtained in one representative experiment out of four). In contrast, other saccharides that are closely related structurally to Δdi4S, at the same concentration, showed no effect on this parameter (data not shown).

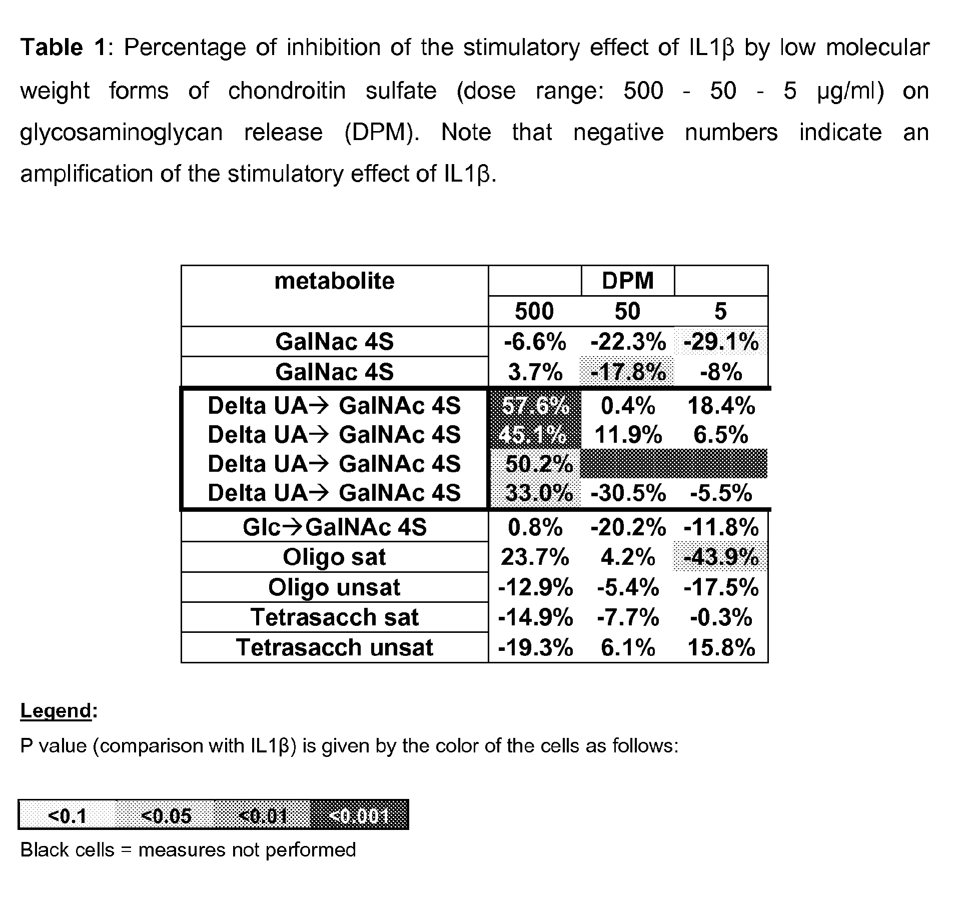

## Claims

1. Use of unsaturated disaccharidic chondroitin sulfate for the preparation of a composition to develop, protect and/or repair connective tissues.

2. Use in accordance with claim 1 wherein the sulfated unsaturated disaccharidic chondroitin sulfate is selected from the group consisting of 4-sulfated unsaturated disaccharidic chondroitin sulfate (Δdi4S), 6-sulfated unsaturated disaccharidic chondroitin sulfate (Δdi6S) and non-sulfated unsaturated disaccharidic chondroitin sulfate (Δdi0S).

3. Use in accordance with one of the preceding claims wherein the composition is a pharmaceutical composition, a food product, a beverage, a food supplement and/or a nutraceutical and in particular selected from the group consisting of milk powder based products; instant drinks; ready-to-drink formulations; nutritional powders; milk-based products, in particular yoghurts or ice cream; cereal products; biscuits; cereal bars; beverages; water; coffee; cappuccino; tea; fruit juices; malt drinks; chocolate flavoured drinks; culinary products; soups; confectionary products; chocolates; topical creams, suppositories, tablets, syrups, and formulations for transdermal applications.

4. Use in accordance with one of the preceding claims to treat or prevent connective tissue diseases.

5. Use in accordance with one of the preceding claims to treat or prevent osteoarthritis or rheumatoid arthritis, and/or the symptoms thereof.

6. Use in accordance with one of the preceding claims to treat or prevent signs of skin ageing such as the appearance of wrinkles.

7. Use in accordance with one of the preceding claims to balance anabolic and catabolic activities of chondrocytes.

8. Use in accordance with one of the preceding claims to slow down or stop cartilage destruction, in particular articular cartilage destruction.

9. Use in accordance with one of the preceding claims to provide pain relief.

10. Use in accordance with one of the preceding claims to improve mobility, in particular to improve joint function.

11. Use in accordance with one of the preceding claims wherein the composition further comprises an aggrecan, a collagen, a chondroitin sulfate and/or a glucosamine or fractions thereof.

12. Use in accordance with one of the preceding claims wherein the composition further comprises an aggrecanase inhibitor or a matrix metalloproteinase inhibitor.

13. Use in accordance with one of the preceding claims wherein the composition further comprises a pain relieving agent, a stabilizing agent, a flavouring agent, a colouring agent, a lubricant, an anti-inflammatory agent, an inhibitor of angiogenesis, a chondroprotective agent a bone anti-resorptive agent and/or an agent increasing bone formation.

14. Use in accordance with one of the preceding claims wherein the composition is intended for humans, in particular for infants, children, adolescents, adults and/or the elderly, or for pets.

15. Use in accordance with one of the preceding claims wherein the 4-sulfated unsaturated chondroitin sulfate is present in the composition in an amount of about 100ng-1g/g dry mass of the composition, and/or is to be administered in a daily amount of about 1ng-100mg /kg body weight of the subject to be treated.
